## Europäisches Patentamt
## European Patent Office
## Office européen des brevets

(11) Veröffentlichungsnummer: **0 092 797**
**B1**

(12)
# EUROPÄISCHE PATENTSCHRIFT

(45) Veröffentlichungstag der Patentschrift:
**11.03.87**

(21) Anmeldenummer: **83103880.7**

(22) Anmeldetag: **20.04.83**

(51) Int. Cl.⁴: **A 61 N 1/04**, A 61 N 1/362,
H 01 B 7/04, H 01 B 7/08,
H 01 B 11/18

(54) **Mehrpolige elektrische Leitung.**

(30) Priorität: **22.04.82 DE 3215021**

(43) Veröffentlichungstag der Anmeldung:
**02.11.83 Patentblatt 83/44**

(45) Bekanntmachung des Hinweises auf die Patenterteilung:
**11.03.87 Patentblatt 87/11**

(84) Benannte Vertragsstaaten:
**DE FR GB IT NL SE**

(56) Entgegenhaltungen:
**EP - A - 0 061 309**
**WO - A - 82/02279**
**DE - A - 2 408 707**
**DE - A - 2 754 342**
**DE - A - 3 019 685**
**DE - A - 3 031 752**
**FR - A - 2 239 000**
**GB - A - 1 119 615**
**GB - A - 1 519 782**
**GB - A - 2 075 744**
**US - A - 4 154 247**

(73) Patentinhaber: **Siemens-Elema AB, Röntgenvägen 2,
S-171 95 Solna 1 (SE)**

(84) Benannte Vertragsstaaten: **SE**

(73) Patentinhaber: **Siemens Aktiengesellschaft Berlin und
München, Witteisbacherplatz 2, D-8000 München 2 (DE)**

(84) Benannte Vertragsstaaten: **DE FR GB IT NL**

(72) Erfinder: **Botvidsson, Lars, Trollvaegen 7,
S-17570 Jaerfaella (SE)**
Erfinder: **Hirschberg, Jakub, Aprilvaegen 3,
S-18344 Täby (SE)**

ACTORUM AG

**Beschreibung**

Die Erfindung betrifft eine mehrpolige Elektrodenanordnung zur Stimulierung von Körpergewebe gemäss dem Oberbegriff des Patentanspruches 1.

Es sind zwei unterschiedliche Typen dieser Leitungen bekannt. Zum Einen eine koaxiale bipolare Leitung mit spiralförmig gewickelten Metalldrähten, die durch einen Isolierschlauch voreinander getrennt und aussen von einem weiteren Isolierschlauch umgeben sind.

Weiterhin ist aus der DE-A 3 031 752 eine Leitung bekannt, bei der die Leiter einzeln isoliert und multifilar zu einer Wendel gewickelt sind.

Da heutzutage vorhandene Stimulatoren, beispielsweise Herzschrittmacher, eine sehr hohe Lebensdauer haben, werden insbesondere bei implantierten Systemen hohe Anforderungen an die Elektrodenleitungen gestellt. Diese sollen geschmeidig sein, um möglichst geringe Organstörungen hervorzurufen. Weiterhin soll der Widerstand klein sein, um den Energieverbrauch niedrig zu halten und damit die Lebensdauer der Batterien zu erhöhen. Die Leitungen müssen eine hohe Biegedauerfestigkeit aufweisen.

Die mechanischen Eigenschaften der Leitungen werden entscheidend durch den Durchmesser der Leitung bestimmt. Dieser liesse sich im Prinzip durch Verringern der Drahtquerschnitte vermindern. Das würde aber auf der anderen Seite zu einem unerwünscht hohen Widerstand führen. Aus diesem Grund muss bei den bekannten koaxialen Leitungen bereits der Durchmesser des äusseren Drahtes grösser sein als der des inneren, da jener länger ist. Ein üblicher erreichbarer Wert für den Durchmesser einer koaxialen Leitung mit Silikongummi als äussere Isolierung ist 2,8 mm.

Mit der aus der DE-A 3 031 752 bekannten Leitung, bei der alle Leiter den selben Abstand von der Achse der Leitung haben, ist zwar bereits der Versuch unternommen worden, den Durchmesser zu verringern. Dabei musste aber eine flachere Steigung der Wendel in Kauf genommen werden, was zu einer verminderten Biegsamkeit und Lebensdauer führt.

Der vorliegenden Erfindung liegt die Aufgabe zugrunde, bei einer Leitung der eingangs genannten Art den Durchmesser wesentlich herabzusetzen und trotzdem die Geschmeidigkeit zu erhöhen und den Widerstand nach Möglichkeit auch noch zu vermindern.

Diese Aufgabe wird erfindungsgemäss durch die Merkmale des Patentanspruches 1 gelöst.

Unter Metallband soll in diesem Zusammenhang auch eine bandförmige Litze aus geflochtenen Drähten verstanden werden.

Bei gleichem Widerstand wie ein Draht ist die Dicke des Metallbandes wesentlich geringer als der Durchmesser des Drahtes, beispielsweise um den Faktor 10. Der Durchmesser der Leitung verringert sich sogar um den doppelten Betrag.

Zusätzlich weist ein wendelförmig gewickeltes Metallband auch noch günstigere Biegeeigenschaften auf als ein entsprechend gewickelter Draht. Durch die Verwendung eines Flachkabels vereinfacht sich zusätzlich auch noch die Herstellung der Leiter. Das Flachkabel braucht nur mit der gewünschten Steigung gewickelt und mit einem äusseren Isoliermantel umgeben zu werden. Besonders vorteilhaft ist es dabei, dass die Isolierung des Flachkabels als Träger für die Metallbänder dient.

Es ist zwar bereits eine unipolare Leitung bekannt, bei der der Leiter aus einem Metallband besteht, jedoch ist dabei ein aus geflochtenen Kunststofffäden bestehender schlauchförmiger Stützkörper im Inneren der Leitung vorgesehen, um ein Zusammenfallen der Leitung beim Biegen zu verhindern und das Einführen eines Führungsdrahtes zu ermöglichen. Wegen dieser Schwierigkeiten und der aufwendigeren Herstellung ist man in der Praxis von derartigen Leitungen wieder abgegangen.

Weiterhin ist aus der DE-A 2 408 707 ein mehrpoliges elektrisches Leitungsorgan bekannt, bei dem ein erster bandförmiger Leiter auf einen elastischen Kern galvanoplastisch aufgebracht ist. Auf einer Zwischenschicht aus elastischem Isoliermaterial folgen dann weitere konzentrisch zu dem ersten liegende Leiter. Den äusseren Abschluss bildet eine Umhüllung, vorzugsweise aus Kunststoff. Hierbei handelt es sich also um ein Leitungsorgan, bei dem blanke Metallbänder oder besser Metallschichten in einem aufwendigen Verfahren auf einen Stützkörper aufgebracht werden.

In einer vorteilhaften Weiterbildung der Erfindung ist vorgesehen, dass Flachkabel mit einer der Anzahl der Leiter entsprechenden Anzahl Metallbändern verwendet werden, wie sie aus der Verdrahtung von Datengeräten oder ähnlichem bekannt sind. Die Herstellung der Leitung wird damit noch einfacher. Gleichzeitig ist der gegenseitige Abstand der Leiter fest vorgegeben.

Vorteilhafterweise sollen die Kanten der verwendeten Flachkabel oder Metallbänder abgerundet sein, um Beschädigungen der Leitung zu vermeiden. Weist die Leitung eine zentrale Öffnung zum Einführen eines Führungsdrahtes auf, so erleichtern die abgerundeten Kanten dessen Einführen.

Insgesamt ergibt sich damit ein Leiter, der die hohen Anforderungen gut erfüllt und eine wesentlich erhöhte Geschmeidigkeit aufweist. Der Durchmesser kann für bipolare Leitungen auf unter 2 mm abgesenkt werden. Dadurch wird bei der Verwendung als Herzschrittmacher-Leitung auch der Blutstrom in der zur Einführung verwendeten Vene erheblich weniger gestört.

Anhand von zwei Figuren werden im folgenden zwei Ausführungsbeispiele der Erfindung näher beschrieben und erläutert.

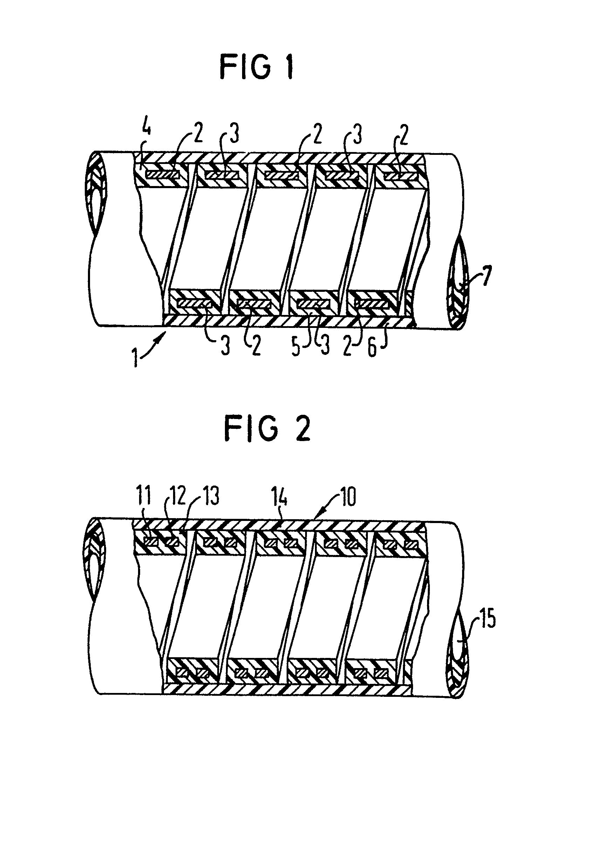

Dabei zeigen Fig. 1 und Fig. 2 im Schnitt zwei bifilar gewickelte bipolare Leitungen.

In Figur 1 ist die Leitung mit 1 bezeichnet. Zwei Leiter 2 und 3 sind jeder für sich vollständig von einer Isolierhülle 4 bzw. 5 umschlossen. Die beiden Leiter sind bifilar wendelförmig gewickelt und aussen von einem Isolierschlauch 6 umgeben. Die

Dicke der für die Leiter verwendeten Metallbänder beträgt ca. 30 µm. Als mögliches Material für die Leiter mit geringem Widerstand und hoher Biegedauerfestigkeit können z.B. extrudiertes Kupfer mit Tantalbeschichtung oder verschiedene Legierungen von Nickel, Kobalt, Chrom, Molybdän und u.U. Eisen verwendet werden. Die Isolierung der Metallbänder kann z.B. aus Silikongummi, Polyäthylen, thermoplastischem Polyurethan oder thermoplastischem Polytetrafluoräthylen bestehen. Für den äusseren, mit Körpergewebe oder Körperflüssigkeit in Berührung kommenden Isolierschlauch eignet sich vorteilhaft Silikongummi oder thermoplastisches Polyurethan.

Fig. 2 zeigt eine Leitung 10, bei der die beiden Leiter 11 bzw. 12 zusammen in einen Isolierkörper 13 eingebettet sind. Metallbänder 11, 12 und Isolierung 13 bilden ein Flachkabel, das z.B. käuflich erwerbbar ist und lediglich wendelförmig gewickelt zu werden braucht. Als äusserer Abschluss dient wieder ein Isolierschlauch 14. Wie bereits aus dem Ausführungsbeispiel gemäss Figur 2 ersichtlich, kann allein durch Verwendung anderer Flachkabel die Anzahl der Leiter variiert werden. Ebenso ist es möglich, mehrere Flachkabel koaxial übereinander zu wickeln. Wegen der geringen Dicke dieser Kabel ist das ohne nennenswerte Steigerung des Leitungsdurchmessers möglich. Auch der multifilare Aufbau einzelner Leiter lässt sich damit einfach verwirklichen.

Beide Leitungen 1 und 10 weisen eine zentrale Öffnung 7 bzw. 15 für einen Führungsdraht auf. Wenn die Anwendung keinen derartigen Draht erfordert, kann auf die zentrale Öffnung verzichtet werden. Ohne den Rahmen der Erfindung zu verlassen, sind auch Kombinationen der beschriebenen Ausführungsbeispiele möglich.

## Patentansprüche

1. Mehrpolige Elektrodenanordnung zur Stimulierung von Körpergewebe mit mindestens zwei gegeneinander isolierten, spiralförmig gewickelten metallischen Leitern, wobei die einzelnen Leiter (2, 3; 11, 12) aus zumindest teilweise mit Isoliermaterial (4, 5; 13) umschlossenem Metallband bestehen, und das Isoliermaterial (4, 5; 13) als Träger für die Metallbänder (2, 3; 11, 12) dient, dadurch gekennzeichnet, dass als mit Isoliermaterial umschlossene Leiter (11, 12) Flachkabel vorgesehen sind.

2. Mehrpolige Elektrodenanordnung nach Anspruch 1, dadurch gekennzeichnet, dass das Flachkabel eine der Anzahl der Leiter (11, 12) entsprechende Anzahl nebeneinander angeordneter Metallbänder enthält.

3. Mehrpolige Elektrodenanordnung nach einem der Ansprüche 1 bis 3, dadurch gekennzeichnet, dass mindestens ein Leiter multifilar aufgebaut ist.

4. Verfahren zur Herstellung einer Elektrodenanordnung nach einem der Ansprüche 1 bis 3, dadurch gekennzeichnet, dass das Flachkabel direkt ohne Stützkörper wendelförmig gewickelt und anschliessend mit einem äusseren Isolierschlauch (14) umhüllt wird.

## Claims

1. A multipole electrode arrangement for stimulating body tissues comprising at least two spirally wound metallic conductors which are insulated from one another, where the individual conductors (2, 3; 11, 12) consist of a metal tape which is at least partially surrounded with insulating material (4, 5; 13) and the insulating material (4, 5; 13) serves as a carrier for the metal tapes (2, 3; 11, 12), characterised in that flat cables surrounded with insulating material are provided as conductors (11, 12).

2. A multipole electrode arrangement as claimed in Claim 1, characterised in that the flat cable comprises a number of adjacently arranged metal tapes, which number corresponds to the number of conductors (11, 12).

3. A multipole electrode arrangement as claimed in one of Claims 1 to 3, characterised in that at least one conductor is of multifilament construction.

4. A process for the production of an electrode arrangement as claimed in one of Claims 1 to 3, characterised in that the flat cable is directly wound in a spiral fashion without any supporting body and is subsequently surrounded with an outer insulating tube (14).

## Revendications

1. Dispositif multipolaire d'électrodes servant à stimuler des tissus corporels et comportant au moins deux fils conducteurs métalliques isolés l'un par rapport à l'autre et enroulés sous forme héliocoïdale, les différents fils conducteurs (2, 3; 11, 12) étant constitués par une bande métallique entourée au moins partiellement par un matériau isolant (4, 5; 13), et le matériau isolant (4, 5; 13) étant utilisé comme support pour les bandes métalliques (2, 3; 11, 12), caractérisé par le fait qu'il est prévu des câbles plats en tant que fils conducteurs (11, 12) enveloppés par un matériau isolant.

2. Dispositif multipolaire d'électrodes suivant la revendication 1, caractérisé par le fait que le câble plat contient un nombre de bandes métalliques disposées côte-à-côte, correspondant au nombre des fils conducteurs (11, 12).

3. Dispositif multipolaire d'électrodes suivant l'une des revendications 1 à 3, caractérisé par le fait qu'au moins un fil conducteur possède une structure multifilaire.

4. Procédé pour fabriquer un dispositif d'électrodes suivant l'une des revendications 1 à 3, caractérisé par le fait qu'on enroule sous forme hélicoïdale le câble plat directement sur un corps de support et qu'on l'enveloppe ensuite avec un tuyau isolant extérieur (14).

# FIG 1

# FIG 2